# EUROPEAN PATENT SPECIFICATION

(11) **EP 4 369 959 B1**
(45) Date of publication and mention of the grant of the patent: **12.03.2025**
(21) Application number: 22737513.6
(22) Date of filing: 14.07.2022
(51) Int. Cl.: A24F 40/40, A24F 40/42, A24F 40/485, A61M 11/04, A61M 15/06, H05B 3/04, H05B 3/42, A24F 40/10

(54) **AEROSOL-GENERATING SYSTEM AND CARTRIDGE FOR AEROSOL-GENERATING SYSTEM WITH SLIDING MECHANISM FOR MECHANICAL SEALING**
AEROSOLERZEUGENDES SYSTEM UND KARTUSCHE FÜR EIN AEROSOLERZEUGENDES SYSTEM MIT GLEITMECHANISMUS ZUR MECHANISCHEN ABDICHTUNG
SYSTÈME DE GÉNÉRATION D'AÉROSOL ET CARTOUCHE POUR SYSTÈME DE GÉNÉRATION D'AÉROSOL DOTÉ D'UN MÉCANISME COULISSANT POUR L'ÉTANCHÉITÉ MÉCANIQUE

(30) Priority: 16.07.2021 EP 21186259
(43) Date of publication of application: 22.05.2024
(73) Proprietor: Philip Morris Products S.A., 2000 Neuchâtel (CH)
(72) Inventor: FREDERICK, Guillaume, 2000 Neuchâtel (CH)
(74) Representative: Reddie & Grose LLP
(86) International application number: PCT/EP2022/069792
(87) International publication number: WO 2023/285618

(56) References cited:
- EP-A1- 3 232 832
- EP-A1- 3 550 998
- WO-A1-2020/039179
- WO-A1-2020/115306
- US-A1- 2019 208 811

## Description

The present disclosure relates to an aerosol-generating system and a cartridge for an aerosol-generating system.

In many known aerosol-generating systems, an aerosol-forming substrate is heated and vaporised to form a vapour. The vapour cools and condenses to form an aerosol. In some aerosol-generating systems, such as electrically heated smoking systems, this aerosol is then inhaled by a user. These aerosol-generating systems often comprise two parts, a cartridge and a control body. The control body contains electronics for controlling the aerosol-generating system. Cartridges for aerosol-generating systems typically comprise an aerosol-forming substrate and a heater for heating the aerosol-forming substrate. A cartridge of this type may include electrical contacts for electrically connecting the heater to the control body. The aerosol-forming substrate is typically a liquid.

Some prior art cartridges have one or more removable or frangible barriers to prevent leakage of the liquid aerosol-forming substrate prior to use. The barriers can be removed or broken when a user is ready to use the cartridge. However, such arrangements have a number of drawbacks. For example, the barriers may be accidentally removed or broken during transport. After such a barrier has been removed or broken, it may not be possible to reseal the cartridge. This may lead to leaking of liquid aerosol-forming substrate prior to first use, or between uses of a multiple use cartridge. Leakage of liquid may interfere with the electrical components of the system, or cause inconvenience for the consumer, or both. Furthermore, such arrangements may be difficult for a consumer to handle, or difficult to manufacture, or both.

WO 2020/115306 A1 discloses an aerosol-generating system comprising a first component and a second component. The first component comprises a first fluidic channel and a first airflow passage and the second component comprises a second fluidic channel and a second airflow passage. A joint rotatably couples the first component to the second component. Rotation, via the joint, of the first component and the second component from a first angle to a second angle relative to one another couples the first fluidic channel to the second fluidic channel.

It would be desirable to address these problems, or at least to provide a viable alternative.

The invention is defined in the appended independent claim 1, to which reference should now be made. Optional features of the invention are defined in dependent claims. According to an aspect of the present disclosure, there is provided a cartridge for an aerosol generating system. The cartridge may comprise a first component comprising a reservoir holding aerosol-forming substrate and an aerosol-forming substrate outlet. The cartridge may comprise a second component comprising an aerosol-forming substrate inlet and an aerosol-generating element. The cartridge may further comprise a sliding mechanism connecting the first component to the second component, and configured to allow the first component to translate from a first position to a second position, relative to the second component. In the first position the aerosol-forming substrate outlet and the aerosol-forming substrate inlet may be not aligned with one another, so that the aerosol-forming substrate cannot pass from the first component to the second component. In the second position the aerosol-forming substrate outlet and the aerosol-forming substrate inlet may be aligned with one another so that the aerosol-forming substrate can pass from the first component to the second component.

Advantageously, the aerosol-forming substrate outlet and the aerosol-forming substrate inlet being not aligned in the first position prevents fluid communication between the reservoir and the aerosol-generating element in the first position. This prevents aerosol-forming substrate from contacting the aerosol-generating element before it is desired. Aerosol-forming substrate in contact with the aerosol-generating element for prolonged periods may lead to corrosion of metal parts of the aerosol-generating element.

The aerosol-forming substrate outlet and the aerosol-forming substrate inlet may be formed on opposing parallel walls of the first and second components respectively. In the first position, the outlet may be not aligned with the inlet and in the second position the outlet may be aligned with the inlet along an axis perpendicular to the parallel walls.

Additionally, the sliding mechanism may be configured to allow the first component to translate only in a lateral direction, orthogonal to the axis perpendicular to the parallel walls.

The first component and the second component may be joined by the sliding mechanism. Advantageously, the first component and the second component contact one another. The first component may comprise at least a first part of the sliding mechanism. The second component may comprise at least a second part of the sliding mechanism. The first part of the sliding mechanism may be configured to engage with the second part of the sliding mechanism. The first part of the sliding mechanism may comprise a protrusion and the second part of the sliding mechanism may comprise a recess that engages with the protrusion. The sliding mechanism may comprise linear sliding guides. The second part of the sliding mechanism may comprise a track and the first part of the sliding mechanism may comprise a guide that engages with the track. Alternatively, the first part of the sliding mechanism may comprise a track and the second part of the sliding mechanism may comprise a guide that engages with the track.

The first component may further comprise a first airflow channel comprising a first air inlet and a first air outlet. The second component may further comprise a second airflow channel comprising a second air inlet and a second air outlet.

In the first position the first air inlet and the second air outlet may be not aligned with one another so that air cannot pass between the second airflow channel and the first airflow channel. In the second position the first air inlet and the second air outlet may be aligned with one another so that air can pass between the second airflow channel and the first airflow channel.

The sliding mechanism may be configured to allow the first component to translate in a lateral direction relative to a longitudinal axis of the first airflow channel.

In the second position, the aerosol-generating element may be in fluid communication with the first airflow channel.

Advantageously, in the first position both the aerosol-forming substrate outlet and inlet and the first air inlet and the second air outlet are not in fluid communication so the aerosol-forming substrate cannot pass between the first and second components.

The cartridge may further comprise a breakable member coupled to the first component and the second component. The breakable member may be configured to resist movement of the first component. The breakable member may be configured to break if sufficient force is applied to break it. Advantageously, the breakable member may prevent accidental translation of the first component before first use. The breakable member may be a frangible seal.

In the first position the aerosol-forming substrate outlet may be sealed by the second component. This prevents leakage of the aerosol-forming substrate into the second component or to outside of the cartridge. Preferably, the cartridge may further comprise a sealing element between the first component and the second component. The sealing element may be configured to prevent leakage of aerosol-forming substrate from the first component when the first component is not in the second position. Advantageously, this prevents loss of aerosol-generating substrate or access to the aerosol-generating substrate by the user, unless the first component is the second position. The sealing element may be fixed to the second component. The sealing element may be a substantially flat sheet. The sealing element may comprise or be formed from an elastomer. For example the sealing element may comprise of be formed from rubber, silicon, or thermoplastic elastomer (TPE). The sealing element may be fixed to the second component on the wall comprising the aerosol-forming substrate inlet. The sealing element may comprise at least one opening to allow fluid transfer across when the first component is in the second position. Preferably, the sealing element is a substantially flat sheet comprising a first opening over the aerosol-forming substrate inlet and a second opening over the second air outlet.

The first component may comprise one or more ribs. The one or more ribs may surround the aerosol-forming substrate outlet. When the first component is in the first position, the aerosol-forming substrate outlet may be sealed by the one or more ribs pressing against the sealing element. Preferably, the one or more ribs may further surround the first air inlet. When the first component is in the first position, the first air inlet may be sealed by the one or more ribs pressing against the sealing element. The one or more ribs may comprise a rib situated between the aerosol-forming substrate outlet and the first air inlet. The rib situated between the aerosol-forming substrate outlet and the first air inlet may advantageously press against the sealing element and seal the aerosol-forming substrate outlet from the first air inlet. This may prevent fluid passing directly between the first air inlet and the aerosol-forming substrate outlet. For example, the rib situated between the aerosol-forming substrate outlet and the first air inlet may press against the sealing element and prevent the aerosol-forming substrate from being transferred from the aerosol-forming substrate outlet directly into the first air inlet.

When the first component is in the second position the one or more ribs may be aligned with the first and second openings in the sealing element. Advantageously, this may allow aerosol-forming substrate to pass from the first component to the second component and air to pass between the second airflow channel and the first airflow channel. Advantageously, in the second position the rib situated between the aerosol-forming substrate outlet and the first air inlet may press against the sealing element between the first and second sealing element openings and seal the aerosol-forming substrate outlet from the first air inlet.

Alternatively, a sealing element may be fixed to the first component and the second component may comprise one or more ribs.

The sliding mechanism may be configured to allow the first component to be translated relative to the second component in a single direction. Unidirectional translation of the first component may advantageously prevent unnecessary back and forth sliding, which could lead to excessive wear and thus damage to the cartridge. The second component may comprise a ratchet mechanism that retains the first component in the second position. Alternatively, the first component may comprise a ratchet mechanism that retains the first component in the second position.

Optionally, the cartridge may comprise a locking mechanism that retains the first component in the second position. This may advantageously prevent accidental sliding of the first component relative to the second component during use.

The first component may further comprise a retractable member that retains the first component in the second position, the retractable member configured to be retracted when in the first position and to be extended into a cavity in the second component when in the second positon.

The second component may comprise a capillary material adjacent to the aerosol-generating element. The capillary material may be in fluid communication with the aerosol-generating element. A capillary material is a material that actively conveys liquid from one end of the material to another. The capillary material delivers aerosol-generating substrate to the aerosol-generating element. The capillary material may have a fibrous or spongy structure. The capillary material preferably comprises a bundle of capillaries. For example, the capillary material may comprise a plurality of fibres or threads or other fine bore tubes. The fibres or threads may be generally aligned to convey aerosol-forming substrate towards the aerosol-generating element. Alternatively, the capillary material may comprise sponge-like or foam-like material. The structure of the capillary material forms a plurality of small bores or tubes, through which the aerosol-forming substrate can be transported by capillary action. The capillary material may comprise any suitable material or combination of materials. Examples of suitable materials are a sponge or foam material, ceramic- or graphite-based materials in the form of fibres or sintered powders, foamed metal or plastics material, a fibrous material, for example made of spun or extruded fibres, such as cellulose acetate, polyester, or bonded polyolefin, polyethylene, terylene or polypropylene fibres, nylon fibres or ceramic. The capillary material may have any suitable capillarity and porosity so as to be used with different aerosol-forming substrate physical properties. The aerosol-forming substrate has physical properties, including but not limited to viscosity, surface tension, density, thermal conductivity, boiling point and vapour pressure, which allow the aerosol-forming substrate to be transported through the capillary medium by capillary action.

The aerosol-generating element may comprise a first side and a second side. The first side may oppose the second side. The first side may be exposed to the second airflow channel and the second side may be exposed to the capillary material. The aerosol-generating element may comprise fluid-permeable material. Fluid may pass through the fluid-permeable material in a liquid or a vapour form. The second airflow channel may be in fluid communication with the capillary material via the fluid permeable aerosol-generating element.

The aerosol-generating element may comprise a heating element. Heating the aerosol-forming substrate may release volatile compounds from the aerosol-forming substrate as a vapour. The vapour may then cool within an airflow (such as within the first airflow channel) to form an aerosol. The heating element may comprise a substantially flat heating element to allow for simple manufacture. Geometrically, the term "substantially flat" heating element is used to refer to a heating element that is in the form of a substantially two dimensional topological manifold. Thus, the substantially flat heating element extends in two dimensions along a surface substantially more than in a third dimension. In particular, the dimensions of the substantially flat heating element in the two dimensions within the surface is at least five times larger than in the third dimension, normal to the surface. An example of a substantially flat heating element is a structure between two substantially imaginary parallel surfaces, wherein the distance between these two imaginary surfaces is substantially smaller than the extension within the surfaces. In some embodiments, the substantially flat heating element is planar. In other embodiments, the substantially flat heating element is curved along one or more dimensions, for example forming a dome shape or bridge shape.

The aerosol-generating element may comprise fluid permeable mesh. The heating element may comprise a plurality of interstices or apertures extending from the second side to the first side and through which fluid may pass. The heating element may comprise a plurality of electrically conductive filaments. The term "filament" is used throughout the specification to refer to an electrical path arranged between two electrical contacts. A filament may arbitrarily branch off and diverge into several paths or filaments, respectively, or may converge from several electrical paths into one path. A filament may have a round, square, flat or any other form of cross-section. A filament may be arranged in a straight or curved manner.

The heating element may be an array of filaments, for example arranged parallel to each other. Preferably, the filaments may form a mesh. The mesh may be woven or non-woven. The mesh may be formed using different types of weave or lattice structures. Alternatively, the electrically conductive heating element consists of an array of filaments or a fabric of filaments. The mesh, array or fabric of electrically conductive filaments may also be characterized by its ability to retain liquid. The electrically conductive filaments may define interstices between the filaments and the interstices may have a width of between 10 micrometres and 100 micrometres. Preferably, the filaments give rise to capillary action in the interstices, so that in use, liquid to be vaporized is drawn into the interstices, increasing the contact area between the heating element and the liquid aerosol-forming substrate.

The electrically conductive filaments may form a mesh of size between 60 and 240 filaments per centimetre (+/- 10 percent). Preferably, the mesh density is between 100 and 140 filaments per centimetres (+/- 10 percent). More preferably, the mesh density is approximately 115 filaments per centimetre. The width of the interstices may be between 100 micrometres and 25 micrometres, preferably between 80 micrometres and 70 micrometres, more preferably approximately 74 micrometres. The percentage of open area of the mesh, which is the ratio of the area of the interstices to the total area of the mesh may be between 40 percent and 90 percent, preferably between 85 percent and 80 percent, more preferably approximately 82 percent.

The electrically conductive filaments may have a diameter of between 8 micrometres and 100 micrometres, preferably between 10 micrometres and 50 micrometres, more preferably between 12 micrometres and 25 micrometres, and most preferably approximately 16 micrometres. The filaments may have a round cross section or may have a flattened cross-section.

The area of the mesh, array or fabric of electrically conductive filaments may be small, for example less than or equal to 50 square millimetres, preferably less than or equal to 25 square millimetres, more preferably approximately 15 square millimetres. The size is chosen such to incorporate the heating element into a handheld system. Sizing of the mesh, array or fabric of electrically conductive filaments less or equal than 50 square millimetres reduces the amount of total power required to heat the mesh, array or fabric of electrically conductive filaments while still ensuring sufficient contact of the mesh, array or fabric of electrically conductive filaments to the liquid aerosol-forming substrate. The mesh, array or fabric of electrically conductive filaments may, for example, be rectangular and have a length between 2 millimetres to 10 millimetres and a width between 2 millimetres and 10 millimetres. Preferably, the mesh has dimensions of approximately 5 millimetres by 3 millimetres.

The aerosol-generating element may be configured to be resistively heated. In other words, the aerosol-generating element may be configured to generate heat when an electrical current is passed though the heating element. The heating element, or portions thereof, may comprise or be formed from any material with suitable electrical and mechanical properties, for example a suitable, electrically resistive material. Suitable materials include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group.

Examples of suitable metal alloys include stainless steel, constantan, nickel-, cobalt-, chromium-, aluminium-, titanium-, zirconium-, hafnium-, niobium-, molybdenum-, tantalum-, tungsten-, tin-, gallium-, manganese- and iron-containing alloys, and super-alloys based on nickel, iron, cobalt, stainless steel, Timetal^{®}, iron-aluminium based alloys and iron-manganese-aluminium based alloys. Timetal^{®} is a registered trade mark of Titanium Metals Corporation 1999 Broadway Suite 4300, Denver Colorado. In composite materials, the electrically resistive material may optionally be embedded in, encapsulated or coated with an insulating material or vice-versa, depending on the kinetics of energy transfer and the external physicochemical properties required. The heating element, or portions thereof, may comprise a metallic etched foil insulated between two layers of an inert material. In that case, the inert material may comprise Kapton^{®}, all-polyimide or mica foil. Kapton^{®} is a registered trade mark of E.I. du Pont de Nemours and Company, 1007 Market Street, Wilmington, Delaware 19898, United States of America. A combination of materials may be used to improve the control of the resistance of the substantially flat heating element. For example, materials with a high intrinsic resistance may be combined with materials with a low intrinsic resistance. This may be advantageous if one of the materials is more beneficial from other perspectives, for example price, machinability or other physical and chemical parameters. Advantageously, a substantially flat filament arrangement with increased resistance reduces parasitic losses. Advantageously, high resistivity heaters allow more efficient use of battery energy.

Preferably, the filaments are made of wire. More preferably, the wire is made of metal, most preferably made of stainless steel.

The electrical resistance of the mesh, array or fabric of electrically conductive filaments of the heater element is preferably between 0.3 and 4 Ohms. More preferably, the electrical resistance of the mesh, array or fabric of electrically conductive filaments is between 0.5 and 3 Ohms, and more preferably about 1 Ohm. Preferably, the electrical resistance is equal or greater than 0.5 Ohms. More preferably, the electrical resistance of the mesh, array or fabric of electrically conductive filaments is between 0.6 Ohms and 0.8 Ohms, and most preferably about 0.68 Ohms.

The heating element may be part of a heater assembly. The heater assembly may comprise the heating element and electrical contact portions, electrically connected to the heating element. The electrical contact portions may be two electrically conductive contact pads. The electrically conductive contact pads may be positioned at an edge area of the heating element. Preferably, the at least two electrically conductive contact pads may be positioned on extremities of the heating element. An electrically conductive contact pad may be fixed directly to electrically conductive filaments of the heating element. An electrically conductive contact pad may comprise a tin patch. Alternatively, an electrically conductive contact pad may be integral with the heating element. The electrical resistance of the mesh, array or fabric of electrically conductive filaments is preferably at least an order of magnitude, and more preferably at least two orders of magnitude, greater than the electrical resistance of the electrical contacts. This ensures that the heat generated by passing current through the heating element is localized to the mesh or array of electrically conductive filaments. It is advantageous to have a low overall resistance for the heating element if the system is powered by a battery. A low resistance, high current system allows for the delivery of high power to the heating element. This allows the heating element to heat the electrically conductive filaments to a desired temperature quickly.

Alternatively, the heating element may comprise a heating plate in which an array of apertures is formed. The apertures may be formed by etching or machining, for example. The plate may be formed from any material with suitable electrical properties, such as the materials described above in relation to filaments of a heating element.

The aerosol-generating element may comprise a susceptor element. In other words, the aerosol-generating element may be configured to operate by inductive heating. In operation, the susceptor may be heated by eddy currents induced in the susceptor. Hysteresis losses may also contribute to the inductive heating.

The aerosol-generating element may atomise the aerosol-forming substrate by a method other than heating. For example, the aerosol-generating element may comprise a vibrating membrane or may force the aerosol-forming substrate through a fine mesh.

The aerosol-forming substrate may be a liquid. The aerosol-forming substrate may be a liquid at room temperature. In that case the reservoir may be described as a liquid reservoir. The aerosol-forming substrate may be in another condensed form, such as a solid at room temperature, or may be in another condensed form, such as a gel, at room temperature. Volatile compounds may be released by heating the aerosol-forming substrate. Volatile compounds may be released by moving the aerosol-forming substrate through passages of a vibratable element. The aerosol-forming substrate may be liquid at room temperature. The aerosol-forming substrate may comprise both liquid and solid components. The liquid aerosol-forming substrate may comprise nicotine. The nicotine containing liquid aerosol-forming substrate may be a nicotine salt matrix. The liquid aerosol-forming substrate may comprise plant-based material. The liquid aerosol-forming substrate may comprise tobacco. The liquid aerosol-forming substrate may comprise a tobacco-containing material containing volatile tobacco flavour compounds, which are released from the aerosol-forming substrate upon heating. The liquid aerosol-forming substrate may comprise homogenised tobacco material. The liquid aerosol-forming substrate may comprise a non-tobacco-containing material. The liquid aerosol-forming substrate may comprise homogenised plant-based material.

The liquid aerosol-forming substrate may comprise one or more aerosol-formers. An aerosol-former is any suitable known compound or mixture of compounds that, in use, facilitates formation of a dense and stable aerosol and that is substantially resistant to thermal degradation at the temperature of operation of the system. Examples of suitable aerosol formers include glycerine and propylene glycol. Suitable aerosol-formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. The liquid aerosol-forming substrate may comprise water, solvents, ethanol, plant extracts and natural or artificial flavours. The liquid aerosol-forming substrate may comprise nicotine and at least one aerosol former. The aerosol former may be glycerine or propylene glycol. The aerosol former may comprise both glycerine and propylene glycol. The liquid aerosol-forming substrate may have a nicotine concentration of between about 0.5% and about 10%, for example about 2%.

The cartridge may further comprise a mouthpiece. The first component may comprise a mouthpiece. Alternatively, the second component may comprise a mouthpiece. The mouthpiece may be connected to the second air outlet. The mouthpiece may be removable. A user may apply a negative pressure to the mouthpiece drawing air from the second air inlet to the first air outlet, allowing aerosol to be drawn by the user. Alternatively, the cartridge may be configured to allow a user to draw directly on the first air outlet.

According to another aspect of the present disclosure, there is provided an aerosol-generating system. The aerosol-generating system may comprise the cartridge of the first aspect, and a control body connected to the cartridge. The control body may comprise a power supply configured to supply power to the aerosol-generating element.

The control body may comprise at least one electrical contact element configured to provide an electrical connection to the aerosol-generating element when the control body is connected to the cartridge. The electrical contact element may be elongate. The electrical contact element may be spring-loaded. The electrical contact element may contact an electrical contact pad in the cartridge.

The control body may comprise control circuitry configured to control a supply of power from the power supply to the aerosol-generating element.

The control circuitry may comprise a microcontroller. The microcontroller is preferably a programmable microcontroller. The control circuitry may comprise further electronic components. The control circuitry may be configured to regulate a supply of power to the aerosol-generating element. Power may be supplied to the aerosol-generating element continuously following activation of the system or may be supplied intermittently, such as on a puff-by-puff basis. The power may be supplied to the aerosol-generating element in the form of pulses of electrical current.

The control body may comprise a power supply configured to supply power to the control circuity and to the aerosol-generating element. Alternatively, the control body may comprise a first power supply configured to supply power to the control circuitry and a second power supply configured to supply power to the aerosol-generating element. The power supply may be a DC power supply. The power supply may be a battery. The battery may be a Lithium based battery, for example a Lithium-Cobalt, a Lithium-Iron-Phosphate, a Lithium Titanate or a Lithium-Polymer battery. The battery may be a Nickel metal hydride battery or a Nickel cadmium battery. The power supply may be another form of charge storage device such as a capacitor. The power supply may require recharging and be configured for many cycles of charge and discharge. The power supply may have a capacity that allows for the storage of enough energy for one or more user experiences; for example, the power supply may have sufficient capacity to allow for the continuous generation of aerosol for a period of around six minutes, corresponding to the typical time taken to smoke a conventional cigarette, or for a period that is a multiple of six minutes. In another example, the power supply may have sufficient capacity to allow for a predetermined number of puffs or discrete activations of the aerosol-generating element.

The control body may be detachably connected to the cartridge. The control body may be connected to the second component of the cartridge. The control body may comprise a connecting portion for engagement with a connection end of the cartridge. The control body may be connected to the cartridge via a screw thread of the cartridge mated with a corresponding screw thread of the control body. Alternatively, the control body may be connected to the cartridge via apertures in the cartridge which form a snap fit connection with corresponding protrusions on the control body. Or, the control body may be connected to the cartridge via apertures in the control body which form a snap fit connection with corresponding protrusions on the cartridge. The second component of the cartridge may be substantially received within a cavity in the control body.

The sliding mechanism may be configured to allow the first component to translate in a lateral direction relative to a longitudinal axis of the control body.

The aerosol-generating system may be a handheld aerosol-generating system configured to allow a user to suck on a mouthpiece to draw an aerosol through the first air outlet. The aerosol-generating system may have a size comparable to a conventional cigar or cigarette. The aerosol-generating system may have a total length between about 25 mm and about 150 mm. The aerosol-generating system may have an external diameter between about 5 mm and about 30mm.

Features of one aspect of the disclosure may be applied to the other aspects of the disclosure.

As used herein, the term "aerosol" refers to a dispersion of solid particles, or liquid droplets, or a combination of solid particles and liquid droplets, in a gas. The aerosol may be visible or invisible. The aerosol may include vapours of substances that are ordinarily liquid or solid at room temperature as well as solid particles, or liquid droplets, or a combination of solid particles and liquid droplets.

As used herein, the term "aerosol-forming substrate" refers to a substrate capable of releasing volatile compounds that can form an aerosol. The volatile compounds may be released by heating or combusting the aerosol-forming substrate.

The aerosol-forming substrate may comprise an aerosol former. As used herein, the term "aerosol-former" refers to any suitable compound or mixture of compounds that, in use, facilitates formation of an aerosol, for example a stable aerosol that is substantially resistant to thermal degradation at the temperature of operation of the system. Suitable aerosol formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate.

The aerosol-forming substrate may comprise nicotine. The aerosol-forming substrate may comprise water. The aerosol-forming substrate may comprise glycerol, also referred to as glycerine, which has a higher boiling point than nicotine. The aerosol-forming substrate may comprise propylene glycol. The aerosol-forming substrate may comprise plant-based material. The aerosol-forming substrate may comprise homogenised plant-based material. The aerosol-forming substrate may comprise tobacco. The aerosol-forming substrate may comprise a tobacco-containing material. The tobacco-containing material may contain volatile tobacco flavour compounds. These compounds may be released from the aerosol forming substrate upon heating. The aerosol-forming substrate may comprise homogenised tobacco material. The aerosol-forming substrate may comprise other additives and ingredients, such as flavourants.

As used herein, the term "liquid aerosol-forming substrate" is used to refer to an aerosol-forming substrate in condensed form. Thus, the "liquid aerosol-forming substrate" may be, or may comprise, one or more of a liquid, gel, or paste. If the liquid aerosol-forming substrate is, or comprises, a gel or paste, the gel or paste may liquidise upon heating. For example, the gel or paste may liquidise upon heating to a temperature of less than 50, 75, 100, 150, or 200 degrees Celsius.

As used herein, the term "heating element" encompasses both an element that is configured to itself raise in temperature when supplied with power and an element that is configured to give rise to an increase in temperature of a coupled component when supplied with power, such as an inductor coil coupled to a susceptor element.

As used herein, a "susceptor element" means a conductive element that heats up when subjected to a changing magnetic field. This may be the result of eddy currents induced in the susceptor element and/or hysteresis losses. Possible materials for the susceptor elements include graphite, molybdenum, silicon carbide, stainless steels, niobium, aluminium and virtually any other conductive elements. Advantageously the susceptor element is a ferrite element. The material and the geometry for the susceptor element can be chosen to provide a desired electrical resistance and heat generation.

Examples will now be further described with reference to the figures in which:
Figure 1 illustrates a schematic cross-sectional view of an aerosol-generating system comprising a first cartridge, with a first component of the cartridge in a first position;
Figure 2 illustrates a schematic cross-sectional view of an aerosol-generating system of Figure 1, with the first component of the cartridge in a second position;
Figure 3 illustrates a cross-sectional view of a cartridge of a second embodiment of the invention with a longitudinal sliding mechanism, with a first component of the cartridge in a first position;
Figure 4 illustrates a cross-sectional view of the cartridge of Figure 3 with a longitudinal sliding mechanism, with the first component of the cartridge in a second position;
Figure 5 illustrates an exploded three-dimensional view of a cartridge of a third embodiment of the invention with an alternative airflow channel and aerosol inlet arrangement, and with a first component of the cartridge in a first position;
Figure 6 illustrates an exploded three-dimensional view of the cartridge of Figure 5, with an alternative airflow channel and aerosol inlet arrangement, and with a first component of the cartridge in a second position;
Figure 7a illustrates a bottom view of the first component of the cartridge of Figures 5 and 6;
Figure 7b illustrates a top view of the second component of the cartridge of Figures 5 and 6;
Figure 8 illustrates a schematic cross-sectional view of the cartridge of Figure 6; and
Figure 9 illustrates a schematic cross-sectional view of an aerosol-generating system of the third embodiment of the invention.

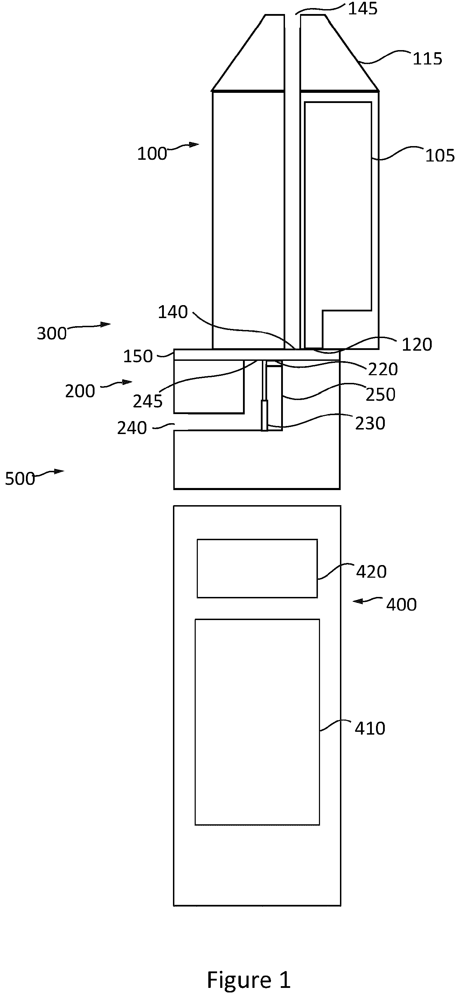

Figure 1 illustrates a schematic cross-sectional view of an aerosol-generating system 500. The aerosol-generating system 500 comprises a cartridge 300 and a control body 400. The cartridge comprises a first component 100 and a second component 200. In this example, the aerosol-generating system 500 is an electrically operated smoking system, often referred to as an e-cigarette system.

The control body 400 is portable and has a size comparable to a conventional cigar or cigarette. The control body 400 comprises a battery 410, such as a lithium iron phosphate battery, and a controller 420 electrically connected to the battery 410.

The cartridge 300 comprises a first component 100 comprising a first airflow channel formed between a first air inlet 140 and a first air outlet 145. A mouthpiece 115 is mounted over the first air outlet 145. A liquid aerosol-forming substrate is held in a reservoir 105. An aerosol-forming substrate outlet 120 in fluid communication with the reservoir 105. The cartridge further comprises a second component 200. The second component 200 comprises an aerosol-forming substrate inlet 220 in fluid communication with a capillary material 250 and an aerosol-generating element 230. In this example, the capillary material 250 has a fibrous structure and is formed from polyester, though any suitable material could be used. The aerosol-generating element 230 comprises a generally planar, fluid permeable mesh heating element, formed from a plurality of filaments. Electrically conductive contact pads are fixed to the aerosol-generating element. When the cartridge 300 is connected to the control body 400, the electrically conductive contact pads are electrically connected to the two electrical contacts in the control body 400. Power is provided to the aerosol-generating element 230 from the battery 410 via this electrical connection. The second component further comprises a second airflow channel comprising a second air inlet 240 and a second air outlet 245. The aerosol-generating element 230 is in fluid communication with the second airflow channel. The aerosol-generating element is positioned downstream of the second air inlet 240 and upstream of the second air outlet 245.

Figure 1 illustrates the cartridge 300 with the first component 100 in the first position. In the first position the aerosol-forming substrate outlet 120 and the aerosol-forming substrate inlet 220 are not aligned with one another so that the aerosol-forming substrate cannot pass between the second component 200 and the first component 100. In particular, the aerosol-forming substrate cannot pass between the aerosol-forming substrate outlet 120 and the aerosol-forming substrate inlet 220 as they are not in fluid communication. As such, the first position may be considered to "seal" the aerosol-forming substrate outlet 120 and the aerosol forming substrate inlet 220 from one another. This inhibits the leakage of the aerosol-forming substrate from the first component 100 into the second component 200, therefore inhibiting leakage into the aerosol-generating element 230. As one option, the system 500 can be purchased by a user in the first position. A breakable member such as a frangible seal may be coupled to the first component 100 and the second component 200. The breakable member could be configured to break if sufficient force is applied. This can inhibit inadvertent translation of the first component 100 relative to the second component 200 until the breakable member is forcibly broken by a user manually translating the first component 100 relative to the second component 200.

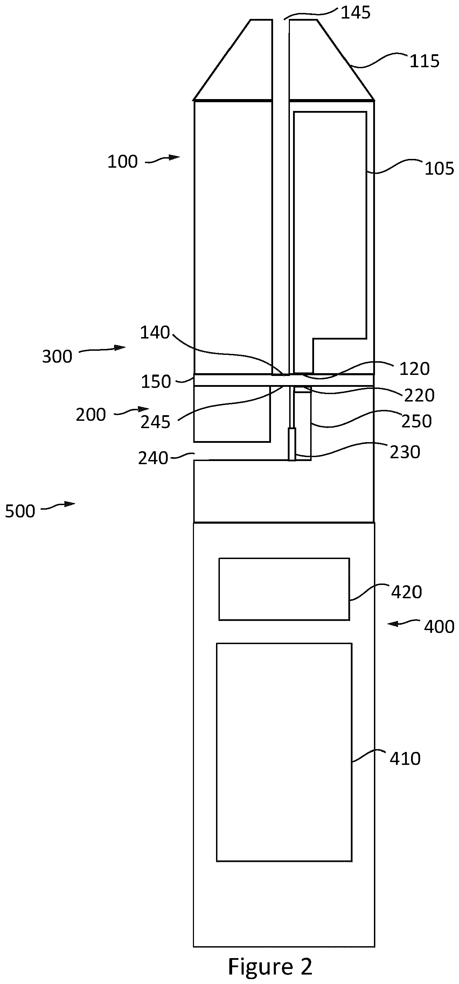

A sliding mechanism 150 connects the first component 100 to the second component 200. The sliding mechanism 150 is configured to allow the first component 100 to translate from a first position (shown in Figure 1) to a second position (shown in Figure 2), relative to the second component 200. As illustrated in Figure 1, the sliding mechanism is configured to allow the first component 100 to translate in a lateral direction relative to a longitudinal axis of the first airflow channel.

The sliding mechanism 150 is configured to allow the first component 100 to be translated relative to the second component 200 in a single direction. The cartridge 300 may further comprise a mechanism configured to retain the first component in the second position such as a ratchet mechanism. The first component 100 is therefore prevented from being translated multiple times by the user. After first use the first component 100 is maintained in the second position. Alternatively, the sliding mechanism 150 could be configured to allow back and forth translation of the first component 100 relative to the second component 200. Therefore, between uses of the aerosol-generating system 500 the user could translate the first component 100 between the first position and the second position.

Figure 2 illustrates a schematic cross-sectional view of an aerosol-generating system comprising the cartridge of Figure 1. In Figure 2 the first component 100 is in a second position and the cartridge 300 is connected to the control body 400.

The control body 400 comprises two electrical contacts which are electrically connected to the battery 410 and configured to provide power to the cartridge 300 via an electrical connection to corresponding contacts in the cartridge 300. This electrical connection is a wired connection and is not shown in Figure 2. In another embodiment, contact points may be configured for inductively heating a susceptor in the cartridge 300. The control body 400 is detachably connected to the cartridge 300. For example, the control body 400 is connected to the cartridge 300 via apertures in the cartridge 300 which form a snap fit connection with corresponding protrusions on the control body 400, this snap fit connection is not shown in Figure 2.

When the first component 100 is in the second position the first air inlet 140 and the second air outlet 245 are aligned with one another so that air can pass between the second component 200 and the first component 100. In the second position the aerosol-forming substrate outlet 120 and the aerosol-forming substrate inlet 220 are aligned with one another. Therefore, aerosol-forming substrate can pass between the aerosol-forming substrate outlet 120 and the aerosol-forming substrate inlet 220. As a result, the aerosol-forming substrate within the reservoir 105 is in fluid communication with the aerosol-generating element 230 within the second component 200. The aerosol-forming substrate within the reservoir 105 is in fluid communication with the aerosol-generating element 230 via the aerosol-forming substrate outlet 120 in the first component, the aerosol-forming substrate inlet 220 in the second component and the capillary material 250. In the second position, the aerosol-generating element 230 is in fluid communication with the first airflow channel, via the second airflow channel. The aerosol-generating element 230 comprises a fluid permeable mesh heating element, comprising a first side and a second side. The first side is exposed the capillary material 250. The second side opposes the first side and is exposed to the second airflow channel. The capillary material 250 is configured to transport aerosol-generating substrate to the aerosol-generating element 230.

The user receives the cartridge 300 in the first position. Prior to first use, the user translates the first component 100 of the cartridge 300 from the first position to the second position, relative to the second component 200. The aerosol-forming substrate outlet 120 and the aerosol-forming substrate inlet 220 are formed on opposing parallel walls of the first 100 and second 200 components respectively. The sliding mechanism 150 is configured to allow the user to translate the first component 100 only in a lateral direction orthogonal to the axis perpendicular to the parallel walls.

The user then connects the cartridge 300 to the control body 400 via apertures in the cartridge 300 which form a snap fit connection with corresponding protrusions on the control body 400. This also electrically connects the cartridge 300 to the control body 400. The battery 410 and controller 420, via the two electrical contacts in the control body 400, become electrically connected to the aerosol-generating element 230, via electrically conductive contact pads in the cartridge 300. Alternatively, the aerosol-generating element 230 comprises a susceptor element. The battery 410 and the controller 420 are electrically connected to an inductor. When the user connects the cartridge 300 to the control body 400 the aerosol-generating element 230 is inductively coupled to the inductor.

Alternatively, the user could translate the first component 100 from the first position to the second position, relative to the second component 200, after connection of the cartridge 300 to the control body 400.

The system shown in Figure 2 shows the aerosol-generating system 500 with the first component 100 of the cartridge 300 in the second position. In this position, the aerosol-forming substrate outlet 120 and the aerosol-forming substrate inlet 220 are aligned with one another so that the aerosol-forming substrate can pass from the first component 100 to the second component 200. The system is configured so that a user can puff or suck on the first air outlet 145 of the cartridge 300 to draw aerosol into their mouth. In operation, when a user puffs on the mouthpiece 115, air is drawn through the second airflow channel from the second air inlet 240, past the aerosol-generating element 230, to the first air inlet 140 and through the first airflow channel to the first air outlet 145. The control circuitry 420 controls the supply of electrical power from the battery 410 to the cartridge 300 when the system is activated. This in turn controls the amount and properties of the vapour produced by the aerosol-generating element 230. The control circuitry 420 may include an airflow sensor and the control circuitry may supply electrical power to the aerosol-generating element 230 when user puffs on the cartridge 300 are detected by the airflow sensor. When a user sucks on the first air outlet 145 of the cartridge 300, the aerosol-generating element 230 is activated and generates a vapour that is entrained in the airflow passing through the first and second airflow channels. The vapour cools to form an aerosol, which is then drawn into the user's mouth through the first air outlet 145.

After use of the aerosol-generating system, the user may translate the first component 100 from the second position back to the first position, relative to the second component 200. Alternatively, the sliding mechanism 150 may be configured to allow the first component 100 to be translated relative to the second component 200 in a single direction.

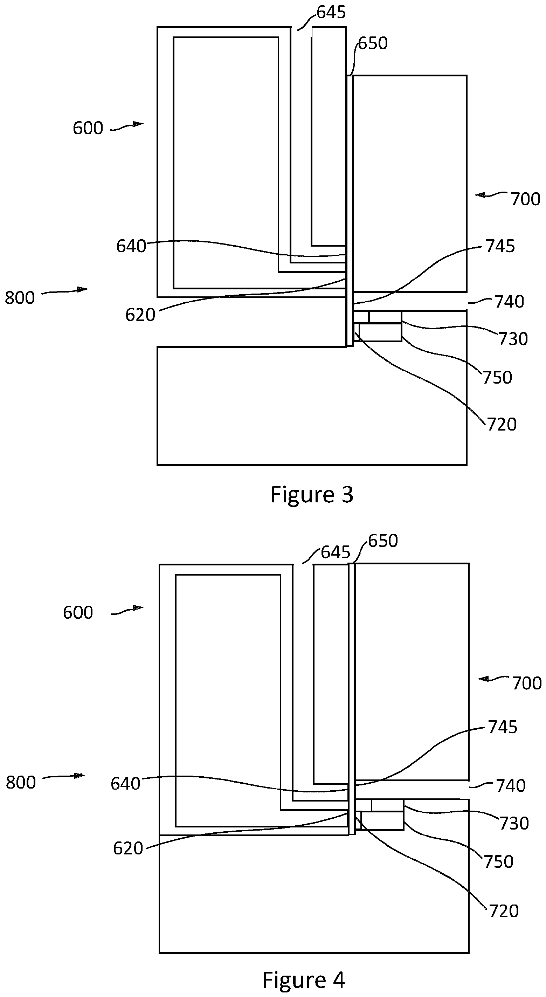

Figure 3 illustrates a cross-sectional view of a cartridge 800 of a second embodiment of the invention that comprises a longitudinally arranged sliding mechanism 650, with a first component 600 of the cartridge 800 in a first position. The cartridge 800 illustrated in Figure 3 includes an aerosol-forming substrate outlet 620 and an aerosol-forming substrate inlet 720 formed on opposing parallel walls of a first 600 and a second 700 component, respectively. In the first position, shown in Figure 3, the outlet 620 is not aligned with the inlet 720 and in the second position the outlet 620 is aligned with the inlet 720 along an axis perpendicular to the parallel walls. The sliding mechanism 650 is configured to allow the first component to translate only in a lateral direction orthogonal to the axis perpendicular to the parallel walls. In this embodiment the parallel walls are parallel to a longitudinal axis of a first airflow channel. Therefore, the sliding mechanism illustrated in Figure 3 is configured to translate the first component in a direction parallel to the longitudinal axis of the first airflow channel.

Figure 4 illustrates the cartridge 800 with a longitudinal sliding mechanism 650, with the first component 600 of the cartridge 800 in the second position. The aerosol-forming substrate outlet 620 and the aerosol-forming substrate inlet 720 are aligned with one another so that the aerosol-forming substrate can pass between the first component 600 and the second component 700.

In this embodiment the aerosol-generating system operates in substantially the same way as the first embodiment. The user translates the first component 600 from the first position to the second position, relative to the second component 700, and then connects the cartridge 800 to a control body. When the first component 600 is in the second position, the user puffs on the first air outlet 645. The aerosol-generating element 730 is activated and generates a vapour of the aerosol-forming substrate. The vapour is entrained in the airflow passing from the second air inlet 740 through the first and second airflow channels. In the airflow channels the vapour cools to form an aerosol, which is then drawn into the user's mouth through the first air outlet 645. After use of the aerosol-generating system, the user may translate the first component 600 from the second position back to the first position, relative to the second component 700.

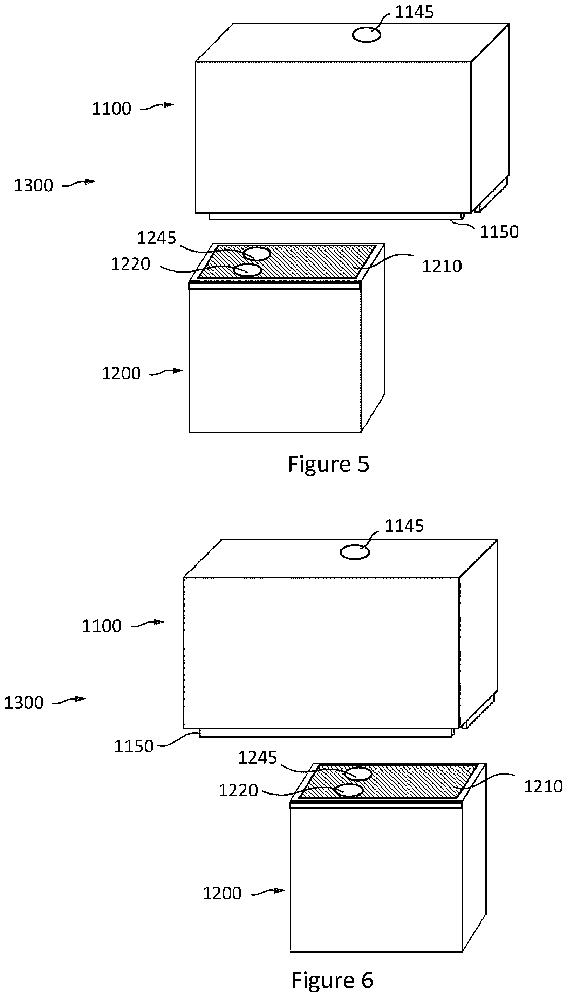

Figure 5 illustrates an exploded three-dimensional view of a cartridge 1300 of a third embodiment of the invention, with a first component 1100 of the cartridge 1300 in a first position. Figure 5 illustrates an alternative airflow channel and aerosol-forming substrate inlet 1220 arrangement to that illustrated in Figure 1. The configuration of a first air inlet 1140, first air outlet 1145, second air inlet 1240 and second air outlet 1245, relative to the aerosol-forming substrate outlet 1120 and the aerosol-forming substrate inlet 1220 is different in the cartridge 1300 of Figure 5 compared to the cartridge of Figure 1. In the cartridge 1300 illustrated in Figure 5, the second air outlet 1245 is adjacent to the aerosol-forming substrate inlet 1220 in a direction perpendicular to the direction in which the sliding mechanism 1150 is configured to allow the first component 1100 to translate, relative to the second component 1200. Figure 5 shows the second component 1200 comprising elastomer sheet sealing element 1210 configured to prevent leakage of aerosol-forming substrate from the first component 1100 when the first component 1100 is not in the second position.

Figure 6 illustrates an exploded three-dimensional view of the cartridge 1300 of Figure 5, with the first component 1100 of the cartridge 1300 in the second position.

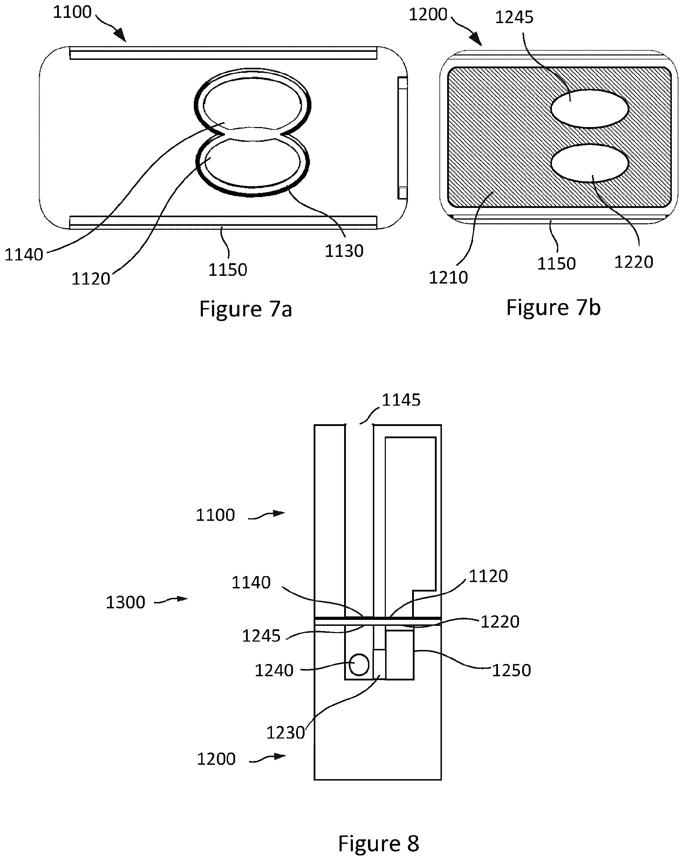

Figure 7a illustrates a bottom view of the first component 1100 of Figures 5 and 6. Figure 7a illustrates the first air inlet 1140 is adjacent to the aerosol-forming substrate outlet 1120 in a direction perpendicular to the direction in which the sliding mechanism 1150 is configured to allow the first component 1100 to translate, relative to the second component 1200. Ribs 1130 surround, and are in-between, the first air inlet 1140 and the first substrate outlet 1120.

Figure 7b illustrates a top view of the second component of Figures 5 and 6. Figure 7b illustrates the second air outlet 1245 and the aerosol-forming substrate inlet 1220. The substantially flat elastomer sheet sealing element 1210 is fixed to the second component 1200.

When the cartridge is assembled and the first component 1100 is in the first position, the sealing element 1210 interacts with the ribs 1130 of the first component. The ribs 1130 press against the sealing element 1210. This seals the first air inlet 1140 and the aerosol-forming substrate outlet 1120 so that fluid cannot pass between the first component 1100 and the second component 1200, or between the first air inlet 1140 and the aerosol-forming substrate outlet 1120. When the first component 1100 is in the second position, the aerosol-forming substrate outlet 1120 is aligned with the first opening in the sealing element 1210 over the aerosol-forming substrate inlet 1220. Furthermore, the first air inlet 1140 is aligned with the second opening in the sealing element 1210 over the second air outlet 1245. Therefore, when the first component 1100 is in the second position, aerosol-forming substrate can pass between the first components 1100 the second component 1200. The rib situated between the aerosol-forming substrate outlet 1120 and the first air inlet 1140 presses against the sealing element 1210 between the first opening in the sealing element and the second opening in the sealing element. This prevents fluid passing directly between the first air inlet 1140 and the aerosol-forming substrate outlet when 1120 when the first component 1100 is in the first position or in the second position.

Figure 8 illustrates a schematic cross-sectional view of the cartridge 1300 of Figure 6. As illustrated in Figure 8, the second air outlet 1245 is adjacent to the aerosol-forming substrate inlet 1220 in a direction perpendicular to the direction in which the sliding mechanism 1150 is configured to allow the first component 1100 to translate, relative to the second component 1200. Furthermore, the first air inlet 1140 is adjacent to the aerosol-forming substrate outlet 1120 in a direction perpendicular to the direction in which the sliding mechanism 1150 is configured to allow the first component 1100 to translate, relative to the second component 1200. The second air inlet 1240 is configured for air to enter the second airflow channel in a direction perpendicular to the direction in which air exits the second airflow channel through the second air outlet 1245.

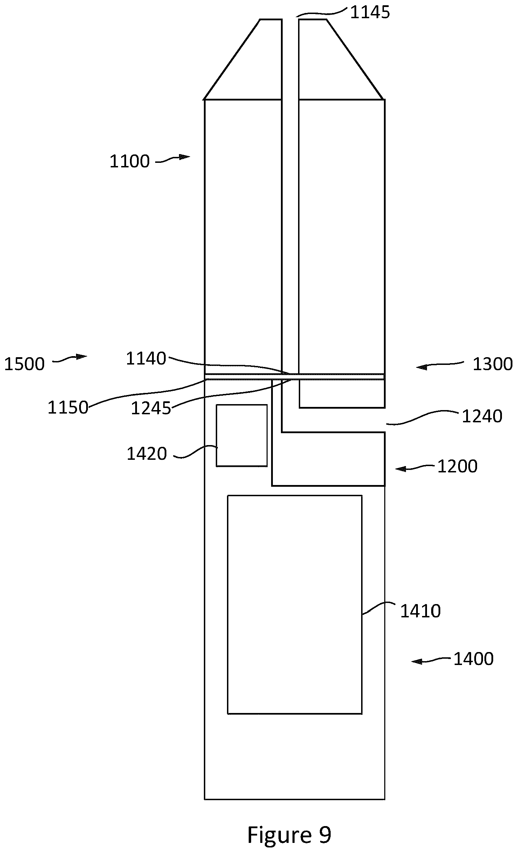

Figure 9 illustrates a schematic cross-sectional view of an aerosol-generating system 1500 of the third embodiment of the invention. The aerosol-generating system 1500 of Figure 9 shows the first component 1100 of the cartridge in the second position relative to the second component 1200. The cartridge 1300 is connected to the control body 1400. The second component 1200 of the cartridge 1300 is substantially received within a cavity in the control body 1400. The control body 1400 comprises a battery 1410, and a controller 1420 electrically connected to the battery 1410. The battery 1410 is configured to provide power to the cartridge 1300 via an electrical connection. This electrical connection is a wired connection and is not shown in Figure 9.

In the third embodiment the aerosol-generating system operates in substantially the same way as the first embodiment. The user via the sliding mechanism 1150, translates the first component 1100 from the first position to the second position, relative to the second component 1200, and then connects the cartridge 1300 to a control body. Subsequently, the user puffs on the first air outlet 1145. The aerosol-generating element 1230 is activated, vaporising the aerosol-forming substrate. The vapour is entrained in the airflow passing from the second air inlet 1240 through the first and second airflow channels. The vapour cools to form an aerosol, which is drawn into the user's mouth through the first air outlet 1145.

For the purpose of the present description and of the appended claims, except where otherwise indicated, all numbers expressing amounts, quantities, percentages, and so forth, are to be understood as being modified in all instances by the term "about". Also, all ranges include the maximum and minimum points disclosed and include any intermediate ranges therein, which may or may not be specifically enumerated herein.

## Claims

1. A cartridge (300, 800, 1300) for an aerosol generating system (500, 1500), the cartridge (300, 800, 1300) comprising:
a first component (100, 600, 1100) comprising a reservoir (105) holding aerosol-forming substrate and an aerosol-forming substrate outlet (120, 620, 1120);
a second component (200, 700, 1200) comprising an aerosol-forming substrate inlet (220, 720, 1220) and an aerosol-generating element (230, 730, 1230); and
a sliding mechanism (150, 650, 1150) connecting the first component (100, 600, 1100) to the second component (200, 700, 1200), and configured to allow the first component (100, 600, 1100) to translate from a first position to a second position, relative to the second component (200, 700, 1200);
wherein in the first position the aerosol-forming substrate outlet (120, 620, 1120) and the aerosol-forming substrate inlet (220, 720, 1220) are not aligned with one another so that the aerosol-forming substrate cannot pass from the first component (100, 600, 1100) to the second component (200, 700, 1200) and in the second position the aerosol-forming substrate outlet (120, 620, 1120) and the aerosol-forming substrate inlet (220, 720, 1220) are aligned with one another so that the aerosol-forming substrate can pass from the first component (100, 600, 1100) to the second component (200, 700, 1200).

2. A cartridge (300, 800, 1300) according to claim 1, wherein the aerosol-forming substrate outlet (120, 620, 1120) and the aerosol-forming substrate inlet (220, 720, 1220) are formed on opposing parallel walls of the first and second components respectively, and wherein in the first position the outlet (120, 620, 1120) is not aligned with the inlet (220, 720, 1220) and in the second position the outlet (120, 620, 1120) is aligned with the inlet (220, 720, 1220) along an axis perpendicular to the parallel walls.

3. A cartridge (300, 800, 1300) according to claim 2, wherein the sliding mechanism (150, 650, 1150) is configured to allow the first component (100, 600, 1100) to translate only in a lateral direction orthogonal to the axis perpendicular to the parallel walls.

4. A cartridge (300, 800, 1300) according to any preceding claim, wherein in the first position the aerosol-forming substrate outlet (120, 620, 1120) is sealed by the second component (200, 700, 1200).

5. A cartridge (300, 800, 1300) according to any preceding claim, wherein the first component (100, 600, 1100) further comprises a first airflow channel comprising a first air inlet (140, 640, 1140) and a first air outlet (145, 645, 1145); and the second component (200, 700, 1200) further comprises a second airflow channel comprising a second air inlet (240, 740, 1240) and a second air outlet (245, 745, 1245).

6. A cartridge (300, 800, 1300) according to claim 5, wherein in the first position the first air inlet (140, 640, 1140) and the second air outlet (245, 745, 1245) are not aligned with one another so that air cannot pass between the second airflow channel and the first airflow channel and in the second position the first air inlet (140, 640, 1140) and the second air outlet (245, 745, 1245) are aligned with one another so that air can pass between the second airflow channel and the first airflow channel.

7. A cartridge (300, 800, 1300) according to claim 5 or 6, wherein the sliding mechanism (150, 650, 1150) is configured to allow the first component (100, 600, 1100) to translate in a lateral direction relative to a longitudinal axis of the first airflow channel.

8. A cartridge (300, 800, 1300) according to any preceding claim, further comprising a breakable member coupled to the first component (100, 600, 1100) and the second component (200, 700, 1200), configured to break if the first component is moved out of the first position.

9. A cartridge (300, 800, 1300) according to any preceding claim, wherein the sliding mechanism is configured to allow the first component (100, 600, 1100) to be translated relative to the second component in a single direction.

10. A cartridge (300, 800, 1300) according to any preceding claim, further comprising a locking mechanism that retains the first component (100, 600, 1100) in the second position.

11. A cartridge according to any preceding claim, wherein the second component (200, 700, 1200) further comprises a capillary material (250, 750, 1250) adjacent to the aerosol-generating element (230, 730, 1230).

12. A cartridge (300, 800, 1300) according to any preceding claim, wherein the aerosol-generating element (230, 730, 1230) comprises fluid permeable mesh.

13. A cartridge (300, 1300) according to any preceding claim, further comprising a mouthpiece (115).

14. An aerosol-generating system (500, 1000) comprising: a cartridge (300, 800, 1300) according to any preceding claim, and a control body (400, 1400) connected to the cartridge (300, 800, 1300), the control body (400, 1400) comprising a power supply configured to supply power to the aerosol-generating element (230, 730, 1230).

15. An aerosol-generating system (500, 1000) according to claim 14, wherein the control body (400, 1400) is directly connected to the second component (200, 700, 1200).

## Patentansprüche

1. Patrone (300, 800, 1300) für ein Aerosolerzeugungssystem (500, 1500), die Patrone (300, 800, 1300) umfassend:
eine erste Komponente (100, 600, 1100), umfassend einen Vorratsbehälter (105), der aerosolbildendes Substrat aufnimmt, und einen Auslass für das aerosolbildende Substrat (120, 620, 1120);
eine zweite Komponente (200, 700, 1200), umfassend einen Einlass für ein aerosolbildendes Substrat (220, 720, 1220) und ein aerosolerzeugendes Element (230, 730, 1230); und
einen Gleitmechanismus (150, 650, 1150), der die erste Komponente (100, 600, 1100) mit der zweiten Komponente (200, 700, 1200) verbindet und dazu eingerichtet ist, um der ersten Komponente (100, 600, 1100) zu ermöglichen, sich von einer ersten Position in eine zweite Position in Bezug auf die zweite Komponente (200, 700, 1200) zu verlagern;
wobei in der ersten Position der Auslass (120, 620, 1120) für das aerosolbildende Substrat und der Einlass (220, 720, 1220) für das aerosolbildende Substrat nicht miteinander ausgerichtet sind, sodass das aerosolbildende Substrat nicht von der ersten Komponente (100, 600, 1100) zu der zweiten Komponente (200, 700, 1200) passieren kann, und in der zweiten Position der Auslass (120, 620, 1120) für das aerosolbildende Substrat und der Einlass (220, 720, 1220) für das aerosolbildende Substrat miteinander ausgerichtet sind, sodass das aerosolbildende Substrat von der ersten Komponente (100, 600, 1100) zu der zweiten Komponente (200, 700, 1200) passieren kann.

2. Patrone (300, 800, 1300) nach Anspruch **1,** wobei der Auslass (120, 620, 1120) für das aerosolbildende Substrat und der Einlass (220, 720, 1220) für das aerosolbildende Substrat an gegenüberliegenden parallelen Wänden jeweils der ersten und zweiten Komponente gebildet sind, und wobei in der ersten Position der Auslass (120, 620, 1120) nicht mit dem Einlass (220, 720, 1220) ausgerichtet ist und in der zweiten Position der Auslass (120, 620, 1120) mit dem Einlass (220, 720, 1220) entlang einer Achse senkrecht zu den parallelen Wänden ausgerichtet ist.

3. Patrone (300, 800, 1300) nach Anspruch 2, wobei der Gleitmechanismus (150, 650, 1150) dazu eingerichtet ist, um der ersten Komponente (100, 600, 1100) zu ermöglichen, sich nur in einer seitlichen Richtung orthogonal zu der Achse senkrecht zu den parallelen Wänden zu verlagern.

4. Patrone (300, 800, 1300) nach einem beliebigen vorhergehenden Anspruch, wobei in der ersten Position der Auslass (120, 620, 1120) für das aerosolbildende Substrat durch die zweite Komponente (200, 700, 1200) abgedichtet ist.

5. Patrone (300, 800, 1300) nach einem beliebigen vorhergehenden Anspruch, wobei die erste Komponente (100, 600, 1100) ferner einen ersten Luftstromkanal umfasst, der einen ersten Lufteinlass (140, 640, 1140) und einen ersten Luftauslass (145, 645, 1145) umfasst; und die zweite Komponente (200, 700, 1200) ferner einen zweiten Luftstromkanal umfasst, der einen zweiten Lufteinlass (240, 740, 1240) und einen zweiten Luftauslass (245, 745, 1245) umfasst.

6. Patrone (300, 800, 1300) nach Anspruch 5, wobei in der ersten Position der erste Lufteinlass (140, 640, 1140) und der zweite Luftauslass (245, 745, 1245) nicht miteinander ausgerichtet sind, sodass Luft nicht zwischen dem zweiten Luftstromkanal und dem ersten Luftstromkanal passieren kann, und in der zweiten Position der erste Lufteinlass (140, 640, 1140) und der zweite Luftauslass (245, 745, 1245) miteinander ausgerichtet sind, sodass Luft zwischen dem zweiten Luftstromkanal und dem ersten Luftstromkanal passieren kann.

7. Patrone (300, 800, 1300) nach Anspruch 5 oder 6, wobei der Gleitmechanismus (150, 650, 1150) dazu eingerichtet ist, der ersten Komponente (100, 600, 1100) zu ermöglichen, sich in einer seitlichen Richtung in Bezug auf eine Längsachse des ersten Luftstromkanals zu verlagern.

8. Patrone (300, 800, 1300) nach einem beliebigen vorhergehenden Anspruch, ferner umfassend ein zerbrechliches Element, das mit der ersten Komponente (100, 600, 1100) und der zweiten Komponente (200, 700, 1200) gekoppelt und dazu eingerichtet ist, zu zerbrechen, wenn die erste Komponente aus der ersten Position bewegt wird.

9. Patrone (300, 800, 1300) nach einem beliebigen vorhergehenden Anspruch, wobei der Gleitmechanismus dazu eingerichtet ist, der ersten Komponente (100, 600, 1100) zu ermöglichen, in Bezug auf die zweite Komponente in einer einzigen Richtung verlagert zu werden.

10. Patrone (300, 800, 1300) nach einem beliebigen vorhergehenden Anspruch, ferner umfassend einen Verriegelungsmechanismus, der die erste Komponente (100, 600, 1100) in der zweiten Position zurückhält.

11. Patrone nach einem beliebigen vorhergehenden Anspruch, wobei die zweite Komponente (200, 700, 1200) ferner ein Kapillarmaterial (250, 750, 1250) angrenzend an das aerosolerzeugende Element (230, 730, 1230) umfasst.

12. Patrone (300, 800, 1300) nach einem beliebigen vorhergehenden Anspruch, wobei das aerosolerzeugende Element (230, 730, 1230) ein fluiddurchlässiges Netz umfasst.

13. Eine Patrone (300, 1300) nach einem beliebigen vorhergehenden Anspruch, ferner umfassend ein Mundstück (115).

14. Aerosolerzeugungssystem (500, 1000), umfassend: eine Patrone (300, 800, 1300) nach einem beliebigen vorhergehenden Anspruch und einen mit der Patrone (300, 800, 1300) verbundenen Regelkörper (400, 1400), wobei der Regelkörper (400, 1400) eine Energieversorgung umfasst, die dazu eingerichtet ist, das aerosolerzeugende Element (230, 730, 1230) mit Energie zu versorgen.

15. Aerosolerzeugungssystem (500, 1000) nach Anspruch 14, wobei der Regelkörper (400, 1400) direkt mit der zweiten Komponente (200, 700, 1200) verbunden ist.

## Revendications

1. Cartouche (300, 800, 1300) pour un système de génération d'aérosol (500, 1500), la cartouche (300, 800, 1300) comprenant :
un premier composant (100, 600, 1100) comprenant un réservoir (105) contenant un substrat formant aérosol et une sortie de substrat formant aérosol (120, 620, 1120) ;
un deuxième composant (200, 700, 1200) comprenant une entrée de substrat formant aérosol (220, 720, 1220) et un élément de génération d'aérosol (230, 730, 1230) ; et
un mécanisme coulissant (150, 650, 1150) raccordant le premier composant (100, 600, 1100) au deuxième composant (200, 700, 1200), et configuré pour permettre au premier composant (100, 600, 1100) de se déplacer en translation d'une première position à une deuxième position, par rapport au deuxième composant (200, 700, 1200) ;
dans laquelle, dans la première position, la sortie de substrat formant aérosol (120, 620, 1120) et l'entrée (220, 720, 1220) ne sont pas alignées les unes avec les autres de sorte que le substrat formant aérosol ne peut pas passer du premier composant (100, 600, 1100) au deuxième composant (200, 700, 1200) et, dans la deuxième position, la sortie de substrat formant aérosol (120, 620, 1120) et l'entrée de substrat formant aérosol (220, 720, 1220) sont alignées l'une avec l'autre de sorte que le substrat formant aérosol peut passer du premier composant (100, 600, 1100) au deuxième composant (200, 700, 1200).

2. Cartouche (300, 800, 1300) selon la revendication 1, dans laquelle la sortie de substrat formant aérosol (120, 620, 1120) et l'entrée de substrat formant aérosol (220, 720, 1220) sont formées sur des parois parallèles opposées des premier et deuxième composants, respectivement, et dans laquelle, dans la première position, la sortie (120, 620, 1120) n'est pas alignée avec l'entrée (220, 720, 1220) et, dans la deuxième position, la sortie (120, 620, 1120) est alignée avec l'entrée (220, 720, 1220) le long d'un axe perpendiculaire aux parois parallèles.

3. Cartouche (300, 800, 1300) selon la revendication 2, dans laquelle le mécanisme coulissant (150, 650, 1150) est configuré pour permettre au premier composant (100, 600, 1100) de se déplacer en translation uniquement dans une direction latérale orthogonale à l'axe perpendiculaire aux parois parallèles.

4. Cartouche (300, 800, 1300) selon l'une quelconque des revendications précédentes, dans laquelle, dans la première position, la sortie de substrat formant aérosol (120, 620, 1120) est fermée de manière étanche par le deuxième composant (200, 700, 1200).

5. Cartouche (300, 800, 1300) selon l'une quelconque des revendications précédentes, dans laquelle le premier composant (100, 600, 1100) comprend en outre un premier canal d'écoulement d'air comprenant une première entrée d'air (140, 640, 1140) et une première sortie d'air (145, 645, 1145) ; et le deuxième composant (200, 700, 1200) comprend en outre un deuxième canal d'écoulement d'air comprenant une deuxième entrée d'air (240, 740, 1240) et une deuxième sortie d'air (245, 745, 1245).

6. Cartouche (300, 800, 1300) selon la revendication 5, dans laquelle, dans la première position, la première entrée d'air (140, 640, 1140) et la deuxième sortie d'air (245, 745, 1245) ne sont pas alignées l'une avec l'autre de sorte que l'air ne peut pas passer entre le deuxième canal d'écoulement d'air et le premier canal d'écoulement d'air et, dans la deuxième position, la première entrée d'air (140, 640, 1140) et la deuxième sortie d'air (245, 745, 1245) sont alignées l'une avec l'autre de sorte que l'air peut passer entre le deuxième canal d'écoulement d'air et le premier canal d'écoulement d'air.

7. Cartouche (300, 800, 1300) selon la revendication 5 ou 6, dans laquelle le mécanisme coulissant (150, 650, 1150) est configuré pour permettre au premier composant (100, 600, 1100) de se déplacer en translation dans une direction latérale par rapport à un axe longitudinal du premier canal d'écoulement d'air.

8. Cartouche (300, 800, 1300) selon l'une quelconque des revendications précédentes, comprenant en outre un organe cassable couplé au premier composant (100, 600, 1100) et au deuxième composant (200, 700, 1200), configuré pour casser si le premier composant est déplacé hors de la première position.

9. Cartouche (300, 800, 1300) selon l'une quelconque des revendications précédentes, dans laquelle le mécanisme coulissant est configuré pour permettre au premier composant (100, 600, 1100) d'être déplacé en translation par rapport au deuxième composant dans une seule direction.

10. Cartouche (300, 800, 1300) selon l'une quelconque des revendications précédentes, comprenant en outre un mécanisme de verrouillage qui retient le premier composant (100, 600, 1100) dans la deuxième position.

11. Cartouche selon l'une quelconque des revendications précédentes, dans laquelle le deuxième composant (200, 700, 1200) comprend en outre un matériau capillaire (250, 750, 1250) adjacent à l'élément de génération d'aérosol (230, 730, 1230).

12. Cartouche (300, 800, 1300) selon l'une quelconque des revendications précédentes, dans laquelle l'élément de génération d'aérosol (230, 730, 1230) comprend un treillis perméable aux fluides.

13. Cartouche (300, 1300) selon l'une quelconque des revendications précédentes, comprenant en outre un embout buccal (115).

14. Système de génération d'aérosol (500, 1000) comprenant une cartouche (300, 800, 1300) selon l'une quelconque des revendications précédentes et un corps de commande (400, 1400) raccordé à la cartouche (300, 800, 1300), le corps de commande (400, 1400) comprenant une alimentation électrique configurée pour alimenter en puissance l'élément de génération d'aérosol (230, 730, 1230).

15. Système de génération d'aérosol (500, 1000) selon la revendication 14, dans lequel le corps de commande (400, 1400) est directement raccordé au deuxième composant (200, 700, 1200).
